# EUROPEAN PATENT APPLICATION

(11) **EP 4 461 197 A1**
(43) Date of publication of application: **13.11.2024**
(21) Application number: 23172889.0
(22) Date of filing: 11.05.2023
(51) Int. Cl.: A61B 1/00, A61B 1/005

(54) **ENDOSCOPE WITH AN ELEVATOR ELEMENT AND CONTROL MODULE FOR THE ENDOSCOPE**

(71) Applicant: Ambu A/S, 2750 Ballerup (DK)
(72) Inventor: CHRISTENSEN, Martin Johst, 2100 København Ø (DK)
(74) Representative: Winter, Brandl - Partnerschaft mbB

(57) **Abstract**

The present disclosure relates to a control module (6) for an endoscope handle (2) with a base (15) configured to be mounted to an inside of the endoscope handle (2), a first steering control assembly comprising a manually operable first steering control element (7a), which is provided for receiving a steering input by a user and is rotatably connected to the base (15) to be rotatable around a control axis (14), and a first wire drum (19a), which is non-rotatably connected to the first steering control element (7a) and is configured for driving a first steering wire (20a), and an elevator control assembly comprising a toothed rack (28) connected or connectable to an elevator wire (12) and an elevator drive gear (27), which forms a toothed portion configured to engage the toothed rack (28), said elevator drive gear (27) being rotatably connected to the base (15) to be rotatable around the control axis (14) at an axial side of the first wire drum (19a), said axial side being axially opposite to the first steering control element (7a).

## Description

The present disclosure relates to a control module for an endoscope and a corresponding endoscope comprising a steering mechanism configured to steer a distal tip unit by bending a bending section of the endoscope, and an elevator element being arranged in an endoscope tip to be movable between a first lowered elevator position and a second raised elevator position for changing a direction of a tool or an instrument inserted through an insertion cord of the endoscope.

### Related Art

Endoscopes and similar specialized instruments such as bronchoscopes, arthroscopes, colonoscopes, laparoscopes, gastroscopes and duodenoscopes are well known from the state of the art and are used for visual examination and diagnosis of hollow organs and body cavities, as well as to assist in surgery, e.g. for a targeted tissue sampling. Basically, a distal tip unit of an endoscope, which is usually connected to a proximal endoscope handle via a bending section and an insertion tube, can be inserted into a hollow organ or body cavity to be investigated with the endoscope. Both reusable and disposable, i.e. single-use, endoscopes are known from the state of the art.

Some endoscopes, in particular duodenoscopes, comprise a distal tip unit having a shovel-like elevator element, a so-called Albarran lever, via which a direction of a tool or an instrument inserted into a patient's body cavity via a working channel of the endoscope can be changed. The elevator element can be raised and lowered in order to set an angle with respect to a longitudinal axis of the distal tip unit at which the tool or instrument exits the distal tip unit into the patient's body cavity. In a raised position of the elevator element, the tool or instrument may e.g. point in a direction essentially perpendicular to the longitudinal axis of the distal tip unit. It is known to control the elevator element via a manually operable element like a control lever provided at the endoscope handle. Elevator mechanics are usually provided via which the manually operable element is connected to a (pull) wire which extends through the insertion cord of the endoscope and which is connected to the elevator element in the distal tip unit.

In related art endoscopes, it is known to invert a motion of the manually operable element by the elevator mechanics such that a clockwise-movement of the manually operable element results in an upward-movement of the elevator element and that an anticlockwise-movement of the manually operable element results in a downward-movement of the elevator element. An operator of related art endoscopes is used to said described motion inversion.

In order to reach the motion inversion it is known from related art endoscopes to connect a slider rod to an operation wheel connected to the manually operable element at a portion of the operation wheel which is essentially/ approximately diametrically opposed to a portion of the operation wheel where the manually operable element is connected to the operation wheel. Expressed differently, the slider rod is not connected to the operation wheel at a side of the same where the manually operable element is provided, but is connected to the operation wheel at an opposed side. Therefore, the slider rod moves around inside a handle housing of the endoscope handle, in particular in a central portion of an accommodation space defined by the handle housing or even in a portion of the handle housing which is close to a surface/ wall which is opposed to a surface! wall at which the manually operable element is provided.

Due to the moving slider rod inside the handle housing, much installation/ accommodation space provided inside the handle housing cannot be used for other parts/ components like valves, which are intended to be accommodated inside the handle housing. Additionally, such a mechanism is disadvantageous since a pulling force applied via a slider rod is not uniform from start to finish of an operating range. This makes it complicated for a user to operate and can lead to miscalculations when operating the elevator element.

Further, solutions comprising a rack and pinion mechanism are known. However, these do not provide control mechanisms that are easy and cost-efficient in manufacturing, can be flexibly combined with various endoscopes providing different functions, and/ or are difficult to handle.

US 5 507 717 A e.g. discloses an endoscope with an elevator at its distal end, wherein the elevator is controlled by a control lever, which is connected to an elevator rod and a slider.

Further, US 5 460 168 A discloses an endoscope with an elevator at the distal tip. The elevator is controlled by a lever. The lever is connected to a control lever gear wheel. The control lever gear wheel has teeth that are in engaging contact with a toothed portion of a connecting member formed as a rack that is movable in an axial direction. The connecting member is connected to a wire that runs to the elevator at the distal tip.

JP 3 349 804 B2 discloses an endoscope with an elevator, which is controlled by a lever. In an embodiment, the lever is connected to a larger gear wheel that is in meshing contact with a smaller gear wheel. A wire is sandwiched between the two gear wheels. A wire end is not connected to any component/ part.

Moreover, CN 111012287 A discloses a single-use endoscope with a forceps lifter at its distal tip. The lifter is activated by a lever. The lever is connected to a first gear wheel that meshes with a second gear wheel. The second gear wheel meshes with a third gear wheel. The third gear wheel is in contact with a rack that moves in an axial direction. The movement of the rack drives a wire that is connected to the forceps lifter. The elevator mechanics provided in CN 111012287 A have the disadvantage that they need much installation space. Moreover, the plurality of meshing engagements provided between the plurality of gear wheels and the toothed rack leads to an increased play in the elevator mechanics, which is also disadvantageous.

### Brief description of the disclosure

In view of the above-described problems it is an object of the present disclosure to provide an endoscope, which shall reduce or avoid the disadvantages of the prior art. In particular, it is an object of the disclosure to provide a (single-use) endoscope having a control module configured for steering a distal tip unit of the endoscope by bending a bending section and for controlling an elevator mechanism, which (single-use) endoscope is easily operable and assemblable, and which control module requires little assembly space.

This object is solved by a control module according to claim 1, an endoscope according to claim 11 and a system according to claim 15. Advantageous aspects of the present disclosure are claimed in the dependent claims and/or are explained below.

In detail, the present disclosure relates to a (modularly installable/ assemblable) control module for an endoscope handle, the control module comprising: a base configured to be mounted to an inside of the endoscope handle, a first steering control assembly comprising a manually operable first steering control element, which is provided for receiving a steering input by a user and is rotatably connected to the base to be rotatable around a control axis, and a first wire drum, which is non-rotatably connected to the first steering control element and is configured for driving a first steering wire, and an elevator control assembly comprising a toothed rack connected or connectable to an elevator wire and an elevator drive gear, which forms a toothed portion configured to (directly or indirectly, preferably directly) engage the toothed rack, said elevator drive gear being rotatably connected to the base to be rotatable around the control axis at an axial side of the first wire drum, said axial side being axially opposite to the first steering control element. I.e. with respect to the axial direction, said elevator drive gear is provided on the one axial side of the first wire drum, and the first steering control element is provided on the other axial side of the first wire drum, so that the first wire drum is axially provided between the first steering control element and the elevator drive gear.

Expressed in other words, a control module for an endoscope is provided, said control module serving as a manual input unit for the user to steer various elements of the endoscope such as a bending section of an insertion cord and an elevator lever of a distal tip unit of said endoscope. Driving elements of the control module such as an elevator drive gear for driving the elevator mechanism and a wire drum for driving a steering wire for bending of the bending section can be preassembled on a base, said base being configured for quick and easy assembly to an endoscope handle, e.g. via bolts, a snap-fit connection, a clamping connection between parts of the handle housing or handle shell, heat staking or the like. Manually operable control and braking elements adapted for user input to drive or lock/ brake the bending mechanism can also be preassembled and can be connected to the driving elements and the endoscope quickly and simply, e.g. via a snap-fit connection. Accordingly, assembly of the control assembly to the endoscope is simple and cost effective. Advantageously, a modularly installable control module is provided which can be assembled into different kinds of endoscopes, such as duodenoscopes, gastroscopes, colonoscopes, etc. That means that the same handle housing, which in the present case is used with an elevator, can also be used for endoscopes without an elevator. I.e. no design change of the handle housing is necessary for endoscopes with or without an elevator. Further, a control stack/ control assembly and a handle housing can be produced in high numbers, which is cost-efficient. Additionally, it is possible to have lower numbers (in total and of different parts) on stock, such that a risk of misassembly is lowered.

Further, since the elevator is operated via a rack and pinion mechanism, a uniform and easily calculable transmission of force to the elevator element is achieved. Thus, user errors are reduced. Further, such a mechanism is very simple and very few parts are needed, thus saving cost for manufacture and assembly. Due to the small number of parts, only few interfaces between such parts need to be provided. Therefore, play in the elevator control assembly can be reduced and a direct and precise control of the elevator control assembly can be achieved. Further, the elevator drive gear is formed at a position, which is arranged axially offset from at least the first wire drum towards an inside of the endoscope handle. Accordingly, an inner radius or diameter of the elevator drive gear is not limited by an outer radius or diameter of said first wire drum. Thus, the elevator drive gear can be made particularly small and an advantageous transmission ratio can be achieved. Often (preferably) the transmission ratio is selected so that the necessary input movement of the control lever is not excessive, i.e. a small angular movement of the elevator control lever provides a large angular movement of the elevator. However sometimes (alternatively preferred) torque is taken into consideration for the transmission ratio. Preferably, an inner radius or diameter of the elevator drive gear is at most 10% larger than an outer radius or diameter of the first wire drum, preferable at most 5% larger, further preferably smaller than the outer radius or diameter of the first wire drum. Also, an axial position of said elevator drive gear can easily be adapted to accommodate functional elements of the endoscope, such that the above described arrangement does not result in a waste of construction space.

In general, in the context of this disclosure, for directions such as "circumferentially", "axially" or "radially", the control axis serves as a reference. Further, "axially outwards" refers to a direction towards an outer side of the endoscope handle in a direction parallel to the control axis and "axially inwards" refers to a direction towards an inside of the endoscope handle in a direction parallel to the control axis, i.e. in a direction opposite to "axially outwards". "Manually operable" means that the steering element is at least partially arranged outside of the endoscope handle such that the user can reach it without disassembly of the endoscope handle. A steering control element is preferably a control knob or hand wheel. The toothed portion may extend along the entire circumference of the elevator drive gear. Preferably, the toothed portion only extends along a portion of the circumference, which will mesh with the toothed rack in a rotational operating range of the elevator drive gear. The base provides a static bearing, being fixedly connected to the endoscope handle and forming various bearing surfaces for movably supporting elements of the control module. The wire drum is configured to wind up or loosen a steering wire in order to pull in or to loosen said steering wire.

Preferably, the control module may have a second steering control assembly comprising a manually operable second steering control element, which is provided for receiving a steering input by a user and is rotatably connected to the base to be rotatable around the control axis (i.e. coaxial with the first steering control assembly), and a second wire drum, which is non-rotatably connected to the second steering control element and is configured for driving a second steering wire.

Expressed in other words, the endoscope - as an alternative to a two-way bending endoscope - may be provided as a four-way bending endoscope, which allows bending of the endoscope in four directions via the (first) steering control element and the second steering control element. Further, the first and second steering control elements can optionally be blocked or brake applied independently from each other by a dedicated braking mechanism described in more detail below. A functional structure of the second steering control assembly can be essentially the same as that of the first steering control assembly. Preferably, the second steering control assembly is inserted or nested inside the first steering control assembly. I.e. the first steering control element and the first wire drum may be arranged axially between the second steering control element and the second wire drum, said first elements being connected by a hollow shaft extending through the first steering control assembly. This achieves a particularly space-efficient structure, which is easily preassembled, thus saving costs.

Preferably, as mentioned above, the control module may further comprise a first braking mechanism adapted to brake the first steering mechanism and/ or a second braking mechanism adapted to brake the second steering mechanism. The first and second braking mechanisms may comprise a friction fit assembly accommodated in the corresponding steering control element and operable via a braking control element supported adjacent to the respective steering control elements.

Preferably, the base forms a casing accommodating at least the first wire drum. The casing may comprise at least a first dome portion or a first capsule portion forming a receptacle for the first wire drum. Optionally, the casing may accommodate the second wire drum. The casing may comprise a second dome portion or a second capsule portion accommodating the second wire drum and being separated by a wall portion from the first dome/ capsule portion. In this manner, each steering control mechanism can be supported securely and precisely, thus allowing a precise and robust steering operation. The casing, optionally the first dome/ capsule portion and/ or the second dome/ capsule portion, may be pot-shaped, with a bottom wall of said pot shape facing axially inwards. An open end of such a pot-shaped first dome/ capsule may lie against an inner surface of the handle housing of the endoscope handle and be attached thereto. An open end of such a pot-shaped second dome/ capsule may lie against an end portion of the first dome/ capsule portion and be attached thereto.

Further preferably, a gear bearing surface for supporting the elevator drive gear in a radial and/ or axial direction is formed at a side of the casing, said side being located axially inwards with respect to the endoscope handle along the control axis. This is a particularly simple way of providing a rotational bearing for the elevator drive gear, reducing material and assembly costs for a separate bearing. In other words, the gear bearing surface may have an axially facing gear bearing portion and/ or a radially facing gear bearing portion. The axially facing gear bearing portion may be formed at an axial end surface of the base. Alternatively, two axially facing portions opposing each other may be formed between two portions of the base, particularly a first dome/ capsule portion accommodating the first wire drum and a second dome/ capsule portion accommodating the second wire drum. The radially facing gear bearing portion may be formed at a circumferential surface of a step or ring-shaped protrusion of the base. In other words, the gear bearing surface may be formed at a side of the base, which is axially opposite to the first wire drum and/ or the second wire drum. That is, the first wire drum and/ or the second wire drum may be separated from the gear bearing surface by a wall portion of the base.

Accordingly, it is particularly preferred that the elevator drive gear is rotatably connected to the base at a position axially inwards of both the first wire drum and the second wire drum. In particular, the elevator drive gear may be attached to an axially innermost end portion of the base. In this manner, a distance between the inner surface of the endoscope housing where the control unit is attached and the elevator drive gear may be maximized. Thus, space for various functional elements of the endoscope is provided between said inner surface and the elevator drive gear.

Alternatively preferred, the elevator drive gear can be rotatably connected to the base at a position axially between the first wire drum and the second wire drum. In this case, the elevator drive gear can be received and supported in a ring-shaped bearing slot formed between the first dome/ capsule portion and the second dome/ capsule portion. No additional securing means for securing the elevator drive gear at the base need to be provided. Thus, costs for securing means and assembly thereof are reduced. An especially precise guidance of the elevator drive gear in said bearing slot can be provided in a simple manner.

The user experience related to operation of the elevator drive may be negatively influenced by friction, and the friction coefficient should preferably be below 0.3, such as in the interval 0.1-0.3. Especially for single use endoscopes it is preferred to use plastic materials, such as thermoplastic materials, and producing the parts by e.g. injection moulding. Friction between components of the same thermoplastic can be considerably higher than for dissimilar materials. Preferably, the gear bearing surface of the base, preferably an entire member forming said gear bearing surface, is made of a material containing a (proprietary) lubricant. A (proprietary) lubricant is a lubricating material such as Teflon particles or a silicon material integrated into a base material to reduce friction between two members that move relative to each other. For example, a material containing a (proprietary) lubricant may be a compound based on a Polycarbonate such as a LNP^{™} LUBRILOY^{™} compound.

Preferably, the base forms a rack bearing surface for slidably supporting the toothed rack to slide in a plane transverse to the control axis, in an extending direction (i.e. longitudinal direction) of the toothed rack. Said rack bearing surface is preferably formed at a position adjacent or near to the above described gear bearing surface. The rack bearing surface may comprise an axially facing rack bearing portion supporting the toothed rack in the axial direction, e.g. at an inner end surface of the first or second dome/ capsule portion. Further, the rack bearing surface may comprise a lateral rack bearing portion supporting the toothed rack laterally along an extension direction of the toothed rack, i.e. in a plane parallel to the control axis. Preferably, the lateral rack bearing portion supports the toothed rack at a side opposite to teeth of the toothed rack. Alternatively or additionally, the lateral rack bearing portion may support the toothed rack at a side of the teeth of the toothed rack, at a flat portion adjacent to the teeth. Alternatively or additionally, the lateral rack bearing portion may support the toothed rack via a rail and groove, one of which is formed in the toothed rack and one of which is formed by the base. The axial rack bearing portion is preferably flush with or formed on the same surface as the gear bearing surface.

Preferably, the elevator drive gear is fixedly connected to, preferably integrally formed with, an elevator control lever. Said elevator control lever extends radially outwards. In particular, said elevator control lever is dimensioned to extend through a slot in the handle housing, such that a grip end of the elevator control lever is manually operable. In particular, the elevator control lever may extend directly radially outwards starting from the elevator drive gear. I.e. the elevator control lever may extend (exclusively) in a plane comprising the elevator drive gear. In this case, the elevator control lever and the elevator drive gear are particularly simple elements which do not need much construction space.

Alternatively, it may be advantageous if the elevator control lever is connected to the elevator drive gear via an axially extending intermediate portion. In this case, the control element can be adapted to a standard endoscope handle where the slot is at a predefined position. Thus, an axial position of the elevator drive wheel can be optimized. The axially extending intermediate portion may be a ring-shaped or cone-shaped portion protruding from the elevator drive gear. Alternatively or additionally, the axially extending intermediate portion may comprise a rod portion connecting the elevator drive gear and the elevator control lever, in particular a rod portion extending diagonally with respect to the control axis.

According to the above-described arrangement, the elevator control lever can be long compared to a radius of the elevator drive gear, thus achieving an advantageous transmission ratio of an operating force, which is applied to the elevator control lever. Preferably, a ratio defined by a distance between a grip end of the elevator control lever and the control axis with respect to the working radius or the elevator drive gear being at least 2, preferably at least 2.3, further preferably at least 2.6.

On the other hand, a toothed rack can be provided with a variable toothing, preferably a variable distance between teeth. In this case, a specific transmission ratio adapted to specific operations can be provided via a simple structure.

Preferably, the control module comprises a module bearing surface formed at a position axially between the first steering control element and the first wire drum, in case of the second steering mechanism also between the second steering control element and the second wire drum. In particular, the module bearing surface may be formed at a radially outer surface of the first steering control assembly, further preferably at the first hollow shaft connecting the first steering control element and the first wire drum. In particular, the module bearing surface is configured to support a housing bearing surface of the endoscope handle. That is, the module bearing surface and the housing bearing surface form a sliding bearing. In other words, the first and/ or second steering control mechanisms are rotatably supported at the endoscope housing via the module bearing surface. In particular, the control module is configured to be supported at the handle housing such that a wall portion of the housing is arranged axially between the first and/ or second wire drums and the elevator drive gear on an inner (one) side and the steering control elements on the outer (other) side. Accordingly, interfaces between the control module and mechanisms of the endoscope are protected in the handle housing while manual control elements are easily reachable for a user.

Preferably, the toothed rack may form a wire connecting portion, particularly a ring-shaped portion adapted to hold a loop formed at a proximal end of the elevator wire. This allows a simple connection between the toothed rack and the elevator wire. Further, the ring-shaped portion may be selectively provided at a distal or a proximal end of the toothed rack. Accordingly, an arrangement of the toothed rack can be adapted to the available space inside the endoscope handle. Optionally, the same toothed rack can be meshed with the elevator drive wheel in either orientation.

Connection elements of the control module and of the endoscope for mounting the control module to the endoscope handle may be standardized, such that various control modules and various endoscopes may be combined. Thus, a constructional platform system can be provided, which allows high flexibility with providing different combinations of control modules with the same endoscope handle or vice versa, while saving costs.

Additionally or alternatively, the object underlying the present disclosure is achieved by an endoscope comprising: a proximal endoscope handle equipped with an above-described control module with at least the first wire drum and the elevator drive gear being accommodated inside the endoscope handle; an insertion cord extending from the endoscope handle and configured to be inserted into a patient's body cavity, the insertion cord comprising an insertion tube, a bending section and a distal tip unit; an elevator mechanism comprising an elevator element, which is accommodated in the distal tip unit and movable between a first lowered elevator position and a second raised elevator position for changing a direction of a tool or an instrument inserted through the insertion cord, and an elevator wire, which is connected to the elevator element to drive said elevator element, extends through the insertion cord and is connected to the toothed rack to be operated via the elevator drive gear; and a first steering wire connected to the bending section, extending through the insertion cord and being operably connected to the first wire drum, said first steering wire being operable to steer the distal tip unit by bending the bending section.

The endoscope may have features discussed above with respect to the control module and its arrangements and interfaces with a corresponding endoscope.

In particular, the endoscope is a single-use endoscope. In such single use endoscopes, costs for manufacture and assembly are minimized. For this, extended use of plastic materials is applied in order to achieve a low cost product based on inexpensive materials. In addition, a structure of such endoscopes is optimize to achieve a low carbon footprint.

Preferably, an axial space is formed between the elevator drive gear and an inner surface of the endoscope handle where the control module is mounted. Further preferably, at least one functional element, in particular at least one valve mounted to the endoscope housing, is at least partially received in said axial space. Expressed in other words, the endoscope handle may comprise at least one valve or other functional element arranged at a position radially adjacent to the control module and axially located between the elevator drive gear and a housing portion of the endoscope handle where the control module is mounted. Thus, it is possible to optimize use of construction space inside the endoscope handle.

Preferably, according to one modification, the toothed rack is positioned at a first radial side of the control module, said first radial side facing the at least one functional element. This is particularly advantageous, since in this case, only two parts (the toothed rack and the elevator drive gear) are necessary in order to drive the elevator element in a specific direction when the elevator control lever is operated in a first predetermined direction. Thus, the corresponding mechanism is particularly simple, cost effective and with minimized play. In particular, the elevator wire may be connected to the elevator drive wheel only through the toothed rack for transmission of force.

Alternatively, the toothed rack may be positioned at another radial side of the control module, said second radial side being opposite to the at least one functional element. In this case, an intermediate gear is preferably provided, which meshes with the elevator drive gear on one side and the toothed rack on another other side. In this manner, the direction in which the elevator element is operated when the elevator control lever is operated in the first predetermined direction can be maintained. This is particularly advantageous if no constructional space is available between the elevator drive gear and the functional element.

Additionally or alternatively, the problem underlying the present disclosure is solved by a system including the above-described endoscope and a monitor connectable to said endoscope.

### Brief description of figures

The following figures illustrate exemplary embodiments of the disclosure. The disclosure is not limited to the embodiments described below. Other embodiments, combinations of embodiments and modifications may be provided within the scope of protection defined by the claims.
Fig. 1 shows a system including a monitor and an endoscope according to the disclosure.
Fig. 2 shows a cross-section of a distal tip unit of the endoscope according to Fig.1.
Fig. 3 shows a cross-sectional view of a portion of the endoscope handle according to a first embodiment of the endoscope according to Fig.1.
Fig. 4 shows a cross-sectional view of a portion of the endoscope handle according to a second embodiment of the endoscope according to Fig.1.
Fig. 5 shows a view of a portion of the interior of the endoscope handle 2 according to the first or second embodiment of the endoscope according to Fig.1.
Figs. 6 and 7 show modifications of a toothed rack of the control module according to the first or second preferred embodiments of the disclosure.
Fig. 8 shows an alternate arrangement of the toothed rack of a control module according to the disclosure.

### Detailed description of preferred embodiments

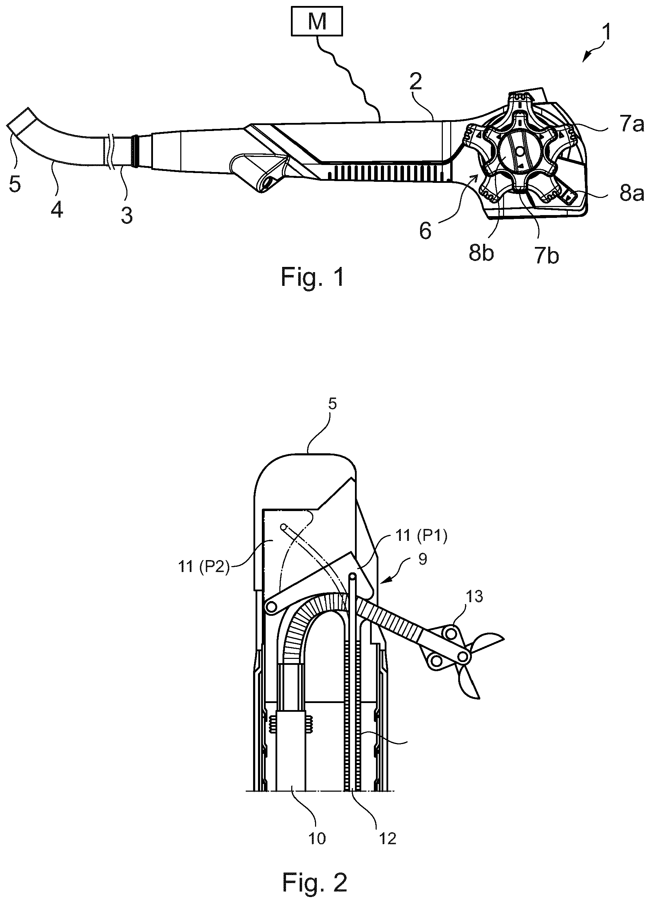

Fig. 1 shows a schematic view of an endoscope 1, which is preferably a single-use endoscope. The endoscope 1 has a proximal endoscope handle 2 with a handle housing and an insertion cord extending distally from the endoscope handle 2. The insertion cord has an insertion tube 3 connected to the endoscope handle 2. The insertion cord includes a bending section 4 connected to a distal end of the insertion tube 3 and a distal tip unit 5 connected to a distal end of the bending section 4. The bending section 4 is configured to perform a bending movement in four different, preferably orthogonal, directions, i.e. two bending planes. This enables a steering of the endoscope 1. The endoscope handle comprises a control module 6 with control elements 7a, 7b, 8a, 8b for controlling the bending section 4, which are described in detail below. Further, Fig. 1 shows a monitor M, which is connected or connectable to the endoscope 1, the monitor M and the endoscope 1 forming a system.

The bending movement in one of the bending planes (in a first and second direction) is controlled via a first steering control assembly including a manually operable first steering control element 7a, in particular formed as a first hand wheel. A first braking mechanism is preferably provided, which has a manually operable first braking control element 8a, in particular formed as a rotatable lever, and is adapted to brake the first steering control assembly, when said first braking control element 8a is operated by a user.

Further, the bending movement in the other one of the bending planes (in a third and fourth direction) is controlled by a second steering control assembly including a manually operable second steering control element 7b, in particular formed as a second hand wheel. A second braking mechanism is preferably provided, which has a manually operable second braking control element 8b, in particular formed as a rotatable knob, and is adapted to brake the second steering control assembly, when said second braking control element 8b is activated by the user. The second steering control assembly and the second braking mechanism may be omitted and the bending section may only be bendable in two directions.

Fig. 2 shows a cross-section of a distal tip unit 5 with an elevator mechanism of the endoscope according to Fig. 1. The distal tip unit 5 forms an internal tip space opening radially to an outside of the distal tip unit 5. I.e. the distal tip unit 5 has a lateral tip opening 9. A working channel 10 extending through the insertion cord opens in the internal tip space. Distally with respect to a distal end of the working channel 10, an elevator element 11 of the elevator mechanism is arranged in the internal tip space. The elevator element 11 is connected to a housing of the distal tip unit 5 via a hinge to be pivotable between a first raised elevator position P1 (depicted in solid lines in Fig. 2) and a second lowered elevator position P2 (depicted in broken lines in Fig. 2). At a position spaced from the hinge, the elevator element 11 is connected to an elevator wire 12 of the elevator mechanism for operating the elevator element 11. The elevator wire 12 is enclosed in an elevator wire sheath and extends through the insertion cord to the endoscope handle 2.

As shown in Fig. 2, when an instrument 13 is inserted through the working channel 10 to the distal tip unit 5, it exits the working channel 10 into the internal tip space and eventually contacts the elevator element 11. By pulling on the elevator wire 12, the elevator element 11 is pivoted to its raised position P1, deflecting the instrument 13 towards the lateral tip opening 9. Thus, the instrument 13 can be pushed out of the lateral tip opening 9 while being guided by the elevator element 11.

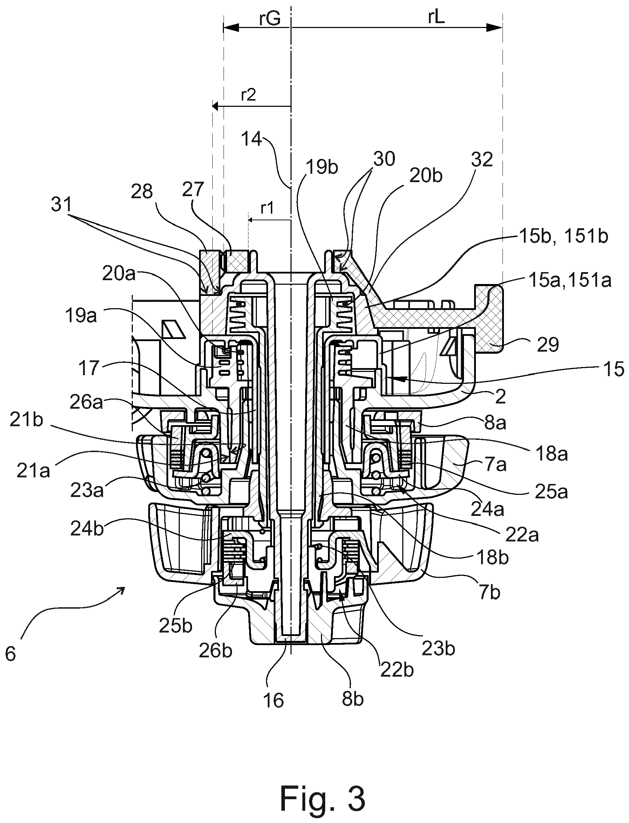

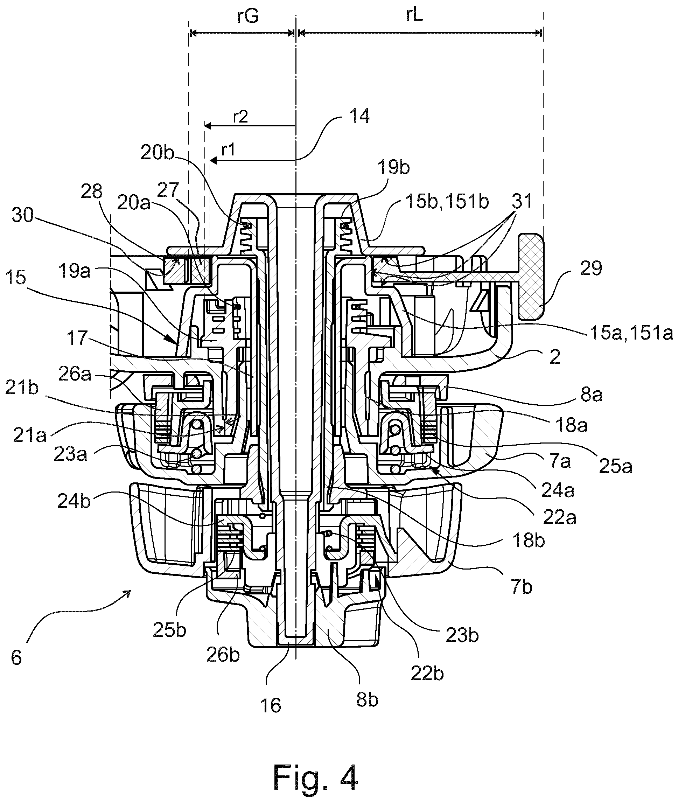

Next, the control module 6 according to a first embodiment of the present disclosure mounted to the endoscope handle 2 is described with reference to Fig. 3, which shows a cross-section of a portion of the endoscope handle (of which only one handle shell part is shown) comprising the control module 6, the cross-section being taken along a control axis 14 of the control module 6. It should be noted that the following description can also be applied to a second embodiment of the control module 6 shown in Fig. 4 and will not be repeated. Differences between the embodiments are described later.

The control module 6 comprises a base 15, which is exchangeably mounted on the endoscope handle 2, in particular to an inside of the endoscope handle 2. The base 15 may have one single base part or two separate base parts, i.e. a first base member 15a and/ or a second base member 15b. The base 15 forms or is fixedly connected to a central shaft 16 and a sleeve-like intermediate support portion 17 coaxially surrounding the central shaft 16. In the present example, the second base member 15b integrally forms the central shaft 16. Further in the present example, the first base member 15a integrally forms intermediate support portion 17. The central shaft 16 defines the control axis 14. The intermediate support portion 17 and the central shaft 16 extend through an opening in the handle housing to an outer side of the endoscope handle 2.

The intermediate support portion 17 supports the first steering control assembly and the first braking mechanism. The central shaft 16 supports the second steering control assembly and the second braking mechanism.

At the outer side of the endoscope handle 2, the first braking control element 8a, the first steering control element 7a, the second steering control element 7b and the second braking control element 8b are arranged in this order starting from an outer side of the endoscope handle 2. In this arrangement, the first braking control element 8a is arranged adjacent to the endoscope handle 2 (an outer surface of the handle housing) and the second braking control element 8b is furthest from the endoscope handle 2 (an outer surface of the handle housing).

The intermediate support portion 17 rotatably supports a first hollow shaft 18a of the first steering control assembly, which is integrally formed with a first wire drum 19a received within the endoscope handle 2. In particular, the first wire drum 19a is received in a first dome portion 151a formed by the base 15, in particular, by the first base member 15a. First steering wires 20a are wound around the first wire drum 19a and extend through the insertion cord to the bending section 4 in order to drive the bending of the bending section 4 in one of the bending planes. Outside of the endoscope handle 2, the first steering control element 7a is non-rotatably connected to the first hollow shaft 18a. Thus, by rotating the first steering control element 7a, the first hollow shaft 18a and the first wire drum 19a are rotated around the control axis 14, pulling or loosening the first steering wire 20a to control the bending section 4.

The central shaft 16 rotatably supports a second hollow shaft 18b, which is integrally formed with a second wire drum 19b received within the endoscope handle 2. In particular, the second wire drum 19b is received in a second dome portion 151b formed by the base 15, in particular by the second base member 15b. Said second dome portion 151b is arranged inwards of the first dome portion 151a, i.e. on a side thereof facing inwards with respect to the handle housing. Second steering wires 20b are wound around the second wire drum 19b and extend through the insertion cord to the bending section 4 in order to drive the bending of the bending section 4 in the other one of the bending planes. The second steering control element 7b, which is arranged outside the endoscope handle 2, is non-rotatably connected to the second hollow shaft 18b. Thus, by rotating the second steering control element 7b, the second wire drum 19b is rotated around the control axis 14, pulling or loosening the second steering wires 20b to control the bending section 4.

At a radially outer surface of the control module, at a position axially between the first and second wire drums 19a, 19b on one side and the first and second steering control elements 7a, 7b on the other side, a module bearing surface 21a is formed as a supporting interface with the handle housing. Preferably, said module bearing surface 21a is formed at a radially outer surface of the first hollow shaft 18a. In particular, the handle housing forms a radially inner housing bearing surface 21b formed at the opening in the handle housing, through which the control module 6 is inserted. The module bearing surface 21a and the radially inner housing bearing surface 21b of the handle housing may be processed (e.g. polished) to form sliding bearing surfaces.

The following description refers to "the braking mechanism" and details first features of the first braking mechanism and second features of the second braking mechanism, which are similar to each other. Similar to the first and second steering control assemblies detailed above, similar features of the first and second braking mechanisms are denoted with reference signs including the letter "a" corresponding to first elements of the first braking mechanism and with reference signs including the letter "b" corresponding to second elements of the second braking mechanism.

The (selectively first or second) braking mechanisms are described in detail below with reference to Fig. 3. In particular, the (selectively first or second) braking mechanisms include the (selectively first or second) braking control element 8a, 8b, a (selectively first or second) braking portion 22a, 22b and (selectively first or second) spring element 23a, 23b. Each steering control element 7a, 7b accommodates the corresponding braking portion 22a, 22b and spring element 23a, 23b. The spring element 23a, 23b applies a preload to the braking portion 22a, 22b.

Each braking portion 22a, 22b includes an axially movable (selectively first or second) carrier 24a, 24b, which on one axial side forms a trough for accommodating the spring element 23a, 23b and supporting it in the axial direction. Axially opposite to the spring element 23a, 23b the carrier 24a, 24b forms a flat supporting surface, where a (selectively first or second) stack of friction discs 25a, 25b is supported. This stack of friction discs 25a, 25b is a friction element according to the disclosure.

In the stack of friction discs 25a, 25b, a number of steering side friction discs and a number of brake side friction discs are alternatingly stacked. The steering side friction discs are non-rotatably and axially slidably engaged with the steering control element 7a, 7b. The brake-side friction discs are non-rotatably and axially slidably connected to the handle housing, e.g. via the central shaft 16 or directly at a portion of the handle housing.

Opposite to the carrier 24a, 24b, a (selectively first or second) plunger 26a, 26b is provided to compress the stack of friction discs 25a, 25b between the carrier 24a, 24b and the flat contact surface, creating a friction fit in the stack of friction discs 25a, 25b and locking the steering control element 7a, 7b with respect to the handle housing. The plunger 26a, 26b is operated via the braking control element 8a, 8b, e.g. via a cam formed on said braking control element 8a, 8b.

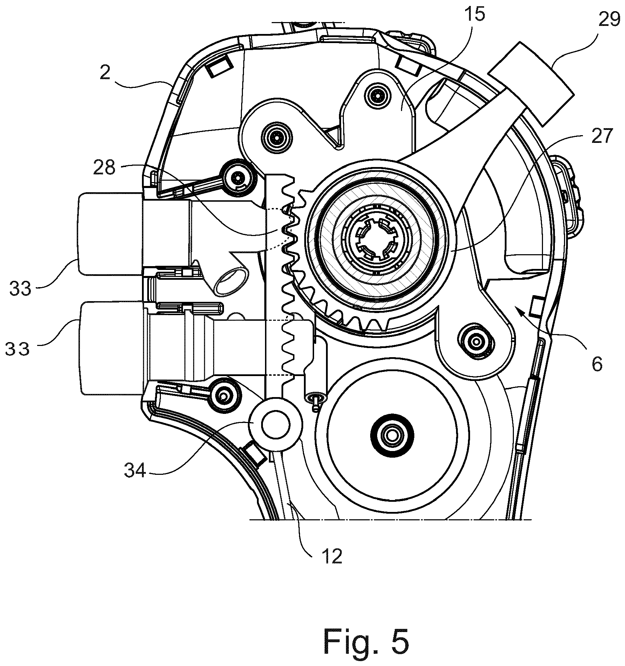

Further, the control module 6 comprises an elevator drive gear 27 and a toothed rack 28. Teeth of the toothed rack 28 engage with teeth of the elevator drive gear 27. The toothed rack 28 is connected or connectable to the elevator wire 12 as shown in Figs. 5 to 8, such that the elevator element 11 is driven via the elevator drive gear 27 and the toothed rack 28. The elevator drive gear 27 is connected to or integrally formed with an elevator control lever 29 extending radially from the elevator drive gear 27. The elevator control lever 29 extends through a slot in the handle housing, such that an operating end of the elevator control lever 29 is arranged at an outer side of the handle housing to be manually operable. A transmission ratio is defined by a ratio rL/rG between an effective gear radius rG of the elevator drive gear 27 and a radial length rL of the elevator control lever 29 with respect to the control axis 14. The elevator drive gear 27 is formed at a position, which is arranged axially offset from at least the first wire drum 19a. Accordingly, an inner radius r1 of the elevator drive gear 27 is not limited by an outer radius r2 of said first wire drum 19a. Thus, the elevator drive gear 27 can be made particularly small and an advantageous transmission ratio can be achieved. Preferably, the transmission ratio is selected so that the necessary input movement of the control lever is not excessive, i.e. a small angular movement of the elevator control lever provides a large angular movement of the elevator. Alternatively preferred in some cases, torque is taken into consideration for the transmission ratio.

The base 15, in particular the second base member 15b, forms a gear bearing surface 30 for supporting the elevator drive gear 27. The gear bearing surface 30 is formed at a position axially inwards with respect to the handle housing, i.e. away from the outer side of the handle housing along an axial direction of the control axis 14. The gear bearing surface 30 may have an axial gear bearing portion. In particular, the axial gear bearing portion is formed at a surface of the base 15, in particular the second base member 15b, facing axially inwards with respect to the handle housing. Preferably, the base 15, in particular the second base member 15b, further forms a rack bearing surface 31 for slidably supporting the toothed rack 28. Preferably, said rack bearing surface 31 is adjacent to, in particular flush with, the axial gear bearing portion. In particular, the rack bearing surface 31 is formed on the same surface as the axial gear bearing portion of the gear bearing surface 30.

According to the first embodiment shown in Fig. 3, the elevator drive gear 27 is arranged axially inwards with respect to/ from both the first wire drum 20a and the second wire drum 20b. In particular, the gear bearing surface 30 and preferably the rack bearing surface 31 are formed at an inner end portion of the base 15, in particular the second base member 15b. In particular, the axial gear bearing portion of the gear bearing surface 30 and preferably the rack bearing surface 31 are formed at an axial end surface of the second dome portion 151b receiving the second wire drum 20b. Further, the base 15, in particular the second base member 15b, may form a ring-shaped protrusion extending axially inwards from the gear bearing surface 30 (the axial gear bearing portion). A radially outer surface of the ring shaped protrusion may form a radial bearing surface portion of the gear bearing surface 30.

According to the second embodiment shown in Fig. 4, the elevator drive gear 27 is arranged axially between the first wire drum 20a and the second wire drum 20b. In particular, the elevator drive gear 27 is sandwiched between a surface of the first dome portion 151a and a surface of the second dome portion 151b formed by the base 15. That is, both the first and second dome portions 151a, 151b (i.e. the first dome portion 151a of the first base member 15a and the second dome portion 151b of the second base member 15b) form gear support surfaces, which face each other in the axial direction across a bearing slot, in which the elevator drive gear 27, is rotatably supported. The first and/ or the second dome portion 151a, 151b may form a collar portion extending radially outwards and forming the corresponding gear bearing surface 30 (the axial gear bearing portion). Further, the first or second dome portion 151a, 151b may form a step or a ring shaped protrusion extending axially towards the respective other one of the first and second dome portions 151a, 151b, a radially outer surface thereof forming the radial bearing surface portion of the gear bearing surface 30.

According to one modification of the first or second embodiment, said modification being exemplarily shown in Fig. 4, the elevator control lever 29 may extend in a plane in which the elevator drive gear 27 is located. In particular, the elevator control lever 29 may extend exclusively radially from the elevator drive gear 27. Alternatively, according to another modification of the first or second embodiment, said modification being exemplarily shown in Fig. 3, a ring-shaped or cone-shaped or rod-shaped axially extending intermediate portion 32 may be provided connecting the elevator drive gear 27 and the elevator control lever 29. Said intermediate portion 32 may extend axially, preferably diagonally with respect to the control axis 14.

Fig. 5 shows a view of a portion of the interior of the endoscope handle 2 according to a first or second embodiment of the endoscope according to Fig. 1, said view being in the direction of the control axis 14 of the control module 6. The base 15 (the first and/ or second base portion 15a, 15b) of the control unit 6 is connected to an inner surface of the handle housing of the endoscope handle 2, preferably via bolts or screws. The endoscope handle 2 includes various functional elements, in particular at least one valve 33, in the present example two valves 33 e.g. for suction and air/ irrigation. The at least one valve 33 is mounted to the handle housing at a position radially adjacent to the control module 6. In particular, the at least one valve 33 is held at a position axially between the inner surface of the handle housing where the control module is mounted and the elevator drive gear 27 and the toothed rack 28. The at least one valve 33 may extend into an axial space formed between the handle housing and the elevator drive gear 27 and/ or the toothed rack 28.

The elevator drive gear 27 may have a toothed portion only at a side facing the toothed rack 28. The elevator control lever 29 is preferably connected to the elevator drive gear 27 at a side diametrically opposite to the toothed portion of the elevator drive gear 27.

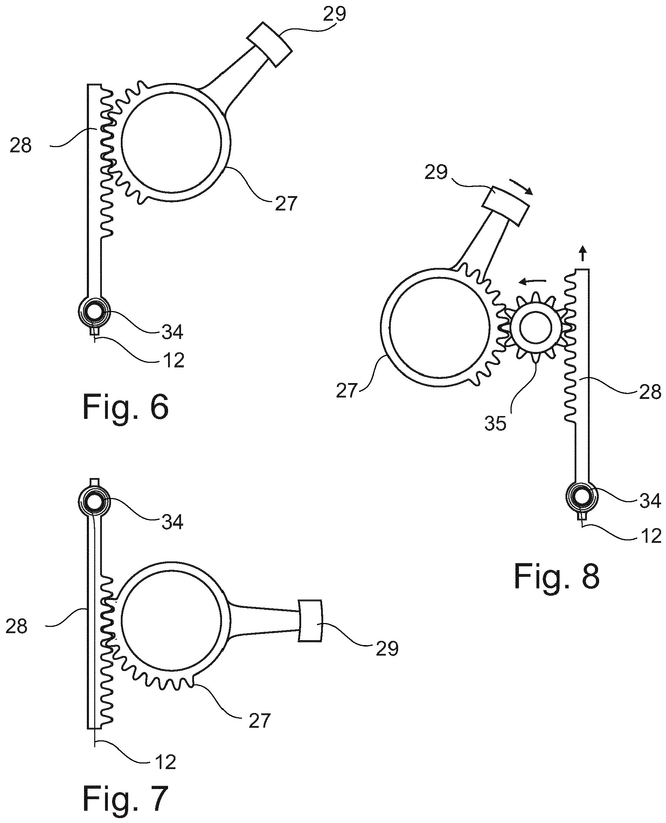

The toothed rack 28 forms at its one end a wire connecting portion 34, which may be ring-shaped, with the elevator wire 12 being wound in a loop around said ring-shaped wire connecting portion 34. Modifications of this wire connecting portion are schematically shown in Fig. 6 and Fig. 7. As shown in Fig. 6, the wire connecting portion 34 can be formed at a distal end (i.e. at a side where the insertion cord is located) of the toothed rack 28. Alternatively, as shown in Fig. 7, the wire connecting portion 34 can be formed at a proximal end (i.e. at a side opposite to the insertion cord) of the toothed rack 28. This alternative position of the wire connecting portion may provide for optimized use of the space inside the handle, or optimized tooth mesh. A Bowden cable will generally be used for transmitting mechanical force to the elevator, in which case the wire will be lead through a wire pipe. To avoid wire slack and possible conflict with mechanisms in the handle it may be advantageous that the wire pipe terminates at a position close to the wire connection portion 34, and having the wire connection portion at the proximal end could give more freedom of design. A further alternative is to provide a wire connection portion 34 at the middle of the toothed rack, i.e. to the side thereof, to have the point where the force acts close to the point where the teeth mesh. Further, as shown in Fig. 6 and 7, an angle of the elevator control lever 29 with respect to the toothed portion may vary.

In the exemplary embodiments shown above, the toothed rack 28 is arranged at a side of the elevator drive gear 27 which faces the at least one valve 33 and meshes directly with the elevator drive gear 27. Alternatively, as schematically shown in Fig. 8, the toothed rack 28 may be arranged at a side of the elevator drive gear 27 opposite to the at least one valve 33. In this case, an intermediate gear 35 is provided between the toothed rack 28 and the elevator drive gear 27, meshing with both of them to transmit a driving force from the elevator drive gear 27 to the toothed rack 28. In this case, the elevator lever is preferably arranged at the same side as the toothed portion, e.g. axially overlapping the toothed portion.

### List of reference numbers

- 1: endoscope
- 2: handle housing
- 3: insertion tube
- 4: bending section
- 5: distal tip unit
- 6: control module
- 7a, 7b: first and second steering control elements
- 8a, 8b: first and second braking control elements
- 9: lateral tip opening
- 10: working channel
- 11: elevator element
- 12: elevator wire
- 13: instrument
- 14: control axis
- 15: base
- 15a: first base portion
- 15b: second base portion
- 151a: first dome portion
- 151b: second dome portion
- 16: central shaft
- 17: intermediate support portion
- 18a, 18b: first and second hollow shafts
- 19a, 19b: first and second wire drums
- 20a, 20b: first and second steering wires
- 21a: radial module bearing surface
- 21b: radial housing bearing surface
- 22a, 22b: first and second braking portions
- 23a, 23b: first and second spring elements
- 24a, 24b: first and second carriers
- 25a, 25b: stacks of friction discs
- 26a, 26b: first and second plunger
- 27: elevator drive gear
- 28: toothed rack
- 29: elevator control lever
- 30: gear bearing surface
- 31: rack bearing surface
- 32: axially extending intermediate portion
- 33: valve
- 34: wire connecting portion
- 35: intermediate gear
- P1: first raised position of elevator element
- P2: second lowered position of elevator element
- rG: effective gear radius of the elevator drive gear
- rL: radial length of the elevator control lever
- r1: inner radius of the elevator drive gear
- r2: outer radius of the first wire drum

## Claims

1. A modularly installable control module (6) for an endoscope handle (2), the control module (6) comprising:
a base (15) configured to be mounted to an inside of the endoscope handle (2);
a first steering control assembly comprising:
a manually operable first steering control element (7a), which is provided for receiving a steering input by a user and is rotatably connected to the base (15) to be rotatable around a control axis (14), and
a first wire drum (19a), which is non-rotatably connected to the first steering control element (7a) and is configured for driving a first steering wire (20a); and
an elevator control assembly comprising:
a toothed rack (28) connected or connectable to an elevator wire (12), and
an elevator drive gear (27), which forms a toothed portion configured to directly or indirectly engage the toothed rack (28), said elevator drive gear (27) being rotatably connected to the base (15) to be rotatable around the control axis (14) at an axial side of the first wire drum (19a), said axial side being axially opposite to an axial side of the first steering control element (7a), so that the first wire drum (19a) is axially provided between the first steering control element (7a) and the elevator drive gear (27).

2. The control module (6) according to claim 1, wherein the base (15) forms a casing accommodating at least the first wire drum (19a), with a gear bearing surface (30) for supporting the elevator drive gear (27) in a radial and/ or axial direction being formed at an axially inwards side of said casing formed by the base (15), the axially inwards side of the casing being axially opposite to an axial side of the first steering control element (7a).

3. The control module (6) according to claim 2, wherein the gear bearing surface (30) of the base (15), preferably an entire member forming said gear bearing surface (30), is made of a material containing a lubricant.

4. The control module (6) according to one of the claims 1 to 3, wherein the base (15) forms a rack bearing surface (31) for slidably supporting the toothed rack (28) to slide in a direction angled, preferably transverse, to the control axis (14).

5. The control module (6) according to one of the claims 1 to 4, further comprising a second steering control assembly comprising
a manually operable second steering control element (7b), which is provided for receiving a steering input by a user and is rotatably connected to the base (15) to be rotatable around the control axis (14), and
a second wire drum (19b), which is non-rotatably connected to the second steering control element (7b) and is configured for driving a second steering wire (20b).

6. The control module (6) according to claim 5, wherein the elevator drive gear (27) is rotatably connected to the base (15) at a position axially inwards of the first wire drum (19a) and the second wire drum (19b).

7. The control module (6) according to claim 5, wherein the elevator drive gear (27) is rotatably connected to the base (15) at a position axially between the first wire drum (19a) and the second wire drum (19b).

8. The control module (6) according to one of the claims 1 to 7, wherein an inner radius (r1) of the elevator drive gear (27) is at most 10% larger than an outer radius (r2) of the first wire drum (19a), preferable at most 5% larger, further preferably smaller than the outer radius (r2) of the first wire drum (19a).

9. The control module (6) according to one of the claims 1 to 8, wherein the elevator drive gear (27) is fixedly connected to, preferably integrally formed with, an elevator control lever (29) extending radially outwards from said elevator drive gear (27), the elevator control lever (29) being preferably connected to the elevator drive gear (27) via an axially extending intermediate portion (32).

10. The control module (6) according to one of the claims 1 to 9, comprising a module bearing surface (21a) formed at a position axially between the first steering control element (7a) and the first wire drum (19a), said module bearing surface (21) preferably being formed at a radially outer surface of the first steering control assembly, said module bearing surface (21a) being configured to support a housing bearing surface (21b) of the endoscope handle (2).

11. An endoscope (1) comprising:
a proximal endoscope handle (2) equipped with a control module (6) according to one of the preceding claims 1 to 10, with at least the first wire drum (19a) and the elevator drive gear (27) being accommodated inside the endoscope handle (2);
an insertion cord extending from the endoscope handle (2) and configured to be inserted into a patient's body cavity, the insertion cord comprising an insertion tube (3), a bending section (4) and a distal tip unit (5);
an elevator mechanism comprising an elevator element (11), which is accommodated in the distal tip unit (5) and movable between a first, raised elevator position (P1) and a second, lowered elevator position (P2) for changing a direction of a tool or an instrument (13) inserted through the insertion cord, and an elevator wire (12), which is connected to the elevator element (11) to drive said elevator element (11), extends through the insertion cord and is connected to the toothed rack (28) to be operated via the elevator drive gear (27); and
a first steering wire (20a) connected to the bending section (4), extending through the insertion cord and being operably connected to the first wire drum (19a), said first steering wire (20a) being operable to steer the distal tip unit (5) by bending the bending section (4).

12. The endoscope according to claim 11, wherein an axial space is formed between the elevator drive gear (27) and an inner surface of the endoscope handle (2) where the control module (6) is mounted, and at least one functional element, preferably at least one valve (33), is least partially received in said axial space.

13. The endoscope according to claim 12, wherein the toothed rack (28) is positioned at a first radial side of the control module (6), said first radial side facing the at least one functional element.

14. The endoscope according to claim 12, wherein the toothed rack (28) is positioned at a second radial side of the control module (6), said second radial side being opposite to the at least one functional element, and an intermediate gear (35) is provided meshing with the elevator drive gear (27) on one side and the toothed rack (28) on another other side.

15. System comprising an endoscope (1) according to one of the claims 11 to 14 and a monitor (M) connectable to the endoscope (1).
